# EUROPEAN PATENT SPECIFICATION

(11) **EP 2 127 667 B1**
(45) Date of publication and mention of the grant of the patent: **23.11.2011**
(21) Application number: 07767787.0
(22) Date of filing: 28.06.2007
(51) Int. Cl.: A61K 38/44, A23L 1/30, A61K 31/19, A61K 31/194, A61K 31/198, A61K 31/7004, A61P 1/02, A61P 31/04

(54) **ORAL DISINFECTANT AND FOOD ADDITIVE COMPRISING THE DISINFECTANT**
MUNDDESINFEKTIONSMITTEL UND NAHRUNGSMITTELZUSATZ MIT DIESEM DESINFEKTIONSMITTEL
DÉSINFECTANT ORAL ET ADDITIF ALIMENTAIRE RENFERMANT CE DÉSINFECTANT

(30) Priority: 28.02.2007 JP 2007049220
(43) Date of publication of application: 02.12.2009
(73) Proprietor: Morinaga Milk Industry Co., Ltd., Minato-ku Tokyo 108-8384 (JP)
(72) Inventor: SHIN, Kouichirou, Zama-shi Kanagawa 228-8583 (JP); HORIGOME, Ayako, Zama-shi Kanagawa 228-8583 (JP); YAMAUCHI, Koji, Zama-shi Kanagawa 228-8583 (JP)
(74) Representative: Albrecht, Thomas
(86) International application number: PCT/JP2007/062993
(87) International publication number: WO 2008/105113

(56) References cited:
- JP-A- 06 500 700
- JP-A- 07 503 707
- JP-A- 59 231 011
- US-A- 4 564 519
- US-A- 5 176 899
- IHALIN RIIKKA ET AL: "Susceptibility of Fusobacterium nucleatum to killing by peroxidase-iodide-hydrogen peroxide combination in buffer solution and in human whole saliva." February 2003 (2003-02), ANAEROBE FEB 2003 LNKD- PUBMED:16887684, VOL. 9, NR. 1, PAGE(S) 23 - 30 , XP002583399 ISSN: 1075-9964 * abstract *
- IHALIN RIIKKA ET AL: "Susceptibilities of different Actinobacillus actinomycetemcomitans strains to lactoperoxidase-iodide-hydrogen peroxide combination and different antibiotics." May 2003 (2003-05), INTERNATIONAL JOURNAL OF ANTIMICROBIAL AGENTS MAY 2003 LNKD- PUBMED:12727076, VOL. 21, NR. 5, PAGE(S) 434 - 440 , XP002583400 ISSN: 0924-8579 * the whole document *
- SHIN K. ET AL.: 'Lactoperoxidase (LPO) Gan'yu Soseibutsu no Kokonai Saikin ni Taisuru Kokin Kassei' NIPPON NOGEI KAGAKUKAI TAIKAI KOEN YOSHISHU no. 3B02P05, March 2007, XP003024638

## Description

### Technical Field

The present invention relates to an oral disinfectant for killing oral bacteria which comprises lactoperoxidase, glucose oxidase, glucose, and a pH adjusting component, and a food additive containing said disinfectant. The oral disinfectant and the food additive comprising the disinfectant according to the present invention have the effect of preventing and/or treating diseases caused by oral bacteria.

### Background Art

Periodontal disease is the second most common oral trouble after dental caries, and is characterized by destroying periodontal tissues such as gingival, periodontal membrane, cementum, and alveolar bone and affecting their functions. The third most common oral trouble after periodontal disease is oral malodor. Oral malodor is classified into pathologic halitosis caused by underlying diseases such as periodontal disease and physiologic halitosis caused by other causes. It has already become apparent that various oral bacteria such as periodontal pathogen are involved in the production of volatile sulfur compounds responsible for breath odor (see, for example, Non-Patent Document 1).

In recent years, there is a concern about aspiration pneumonia resulting from aspiration of saliva and food (i.e., accidental entry of a liquid or solid into the trachea during swallowing) in elderly persons and those who require nursing care. In this regard, it has been pointed out that there is a possibility that oral bacteria are involved in the occurrence of aspiration pneumonia. Therefore, keeping good oral hygiene can be an important way for elderly persons and those who require nursing care to protect their life. For this reason, there is a growing awareness of the importance of oral care (see, for example, Non-Patent Document 2).

Among important oral bacteria affecting oral hygiene, gram-negative bacteria such as Actinobacillus actinomycetemcomitans and Fusobacterium nucleatum are known as periodontal pathogen causing periodontal disease. Particularly, Actinobacillus actinomycetemcomitans is responsible for aggressive periodontitis characterized by rapidly progressing toward severe periodontitis. In the oral cavity, various oral bacteria other than these typical pathogenic bacteria such as periodontal pathogen also live and form an oral bacterial flora.

Lactoperoxidase, a kind of milk protein, is an oxidoreductase contained not only in mammalian milks but also in secretions such as saliva, tear, and airway mucus, and can be industrially produced on a large scale from cow's milk (see, for example, Patent Documents 1 and 2). It is known that such lactoperoxidase catalyzes the production of hypothiocyanite in the presence of hydrogen peroxide and thiocyanate so that strong antibacterial activity is exhibited. Such an antibacterial system (i.e., a system in which lactoperoxidase catalyzes the production of hypothiocyanite in the presence of hydrogen peroxide and thiocyanate so that strong antibacterial activity is exhibited) is generally called lactoperoxidase system.

It is generally known that such a lactoperoxidase system does not exhibit antibacterial activity and bactericidal activity against gram-positive bacteria such as cariogenic bacteria, but exhibits antibacterial activity against some gram-negative bacteria (many of periodontal pathogen are gram negative) (see, for example, Patent Document 3). However, it has been reported that the above-mentioned bacteria such as Actinobacillus actinomycetemcomitans and Fusobacterium nucleatum known as causative bacteria of periodontal disease are less sensitive to the lactoperoxidase system despite the fact that they are gram negative, that is, the lactoperoxidase system does not have a sufficient bactericidal activity against Actinobacillus actinomycetemcomitans etc (see, for example, Non-Patent Document 3). Therefore, in order to achieve a sufficient bactericidal activity against Actinobacillus actinomycetemcomitans and Fusobacterium nucleatum, a lactoperoxidase system using iodine ions instead of thiocyanate has been proposed (see, for example, Non-Patent Document 4).

Thiocyanate and its salts for use in the above-described lactoperoxidase system are not generally approved as food additives, and there is no prospect that they will be approved as food additives. Further, iodine ions and iodine compounds proposed as substitutes for thiocyanate are not approved as food additives either, and there is no probability that they will be approved as food additives.
Patent Document 1: Japanese Patent Application Laid-Open No. 05-41981
Patent Document 2: WO 2005/078078
Patent Document 3: Japanese Patent Publication No. 04-25924

Non-Patent Document 1: "Clinical Guideline For Halitosis", K. Yaegaki ed., Quintessence Pub., Tokyo, 2000, pp. 13-26
Non-Patent Document 2: Gerodontology, U.K., vol. 23, 2006, pp. 55-59
Non-Patent Document 3: Journal of Periodontal Research, Denmark, vol. 33, 1998, pp. 421-427
Non-Patent Document 4: International Journal of Antimicrobial Agents, Netherland, vol. 21, 2003, pp. 434-440

U.S. patent 5,176,899 describes a stabilized aqueous dentifrice composition capable of producing or, in the presence of saliva, leading to the production of antimicrobially effective concentrations of hypothiocyanite ions (OSCN-). The composition contains both an oxidoreductase enzyme and its specific substrate, for the purpose of producing hydrogen peroxide of at least the minimum effective concentration. The composition has a pH of not less than 5.5.

Ihalin et al. (Anaerobe, vol. 9, pp 23-30 (2003)) describe the susceptibility of Fusobacterium nucleatum to killing by peroxidase-iodide-hydrogen peroxide combination in buffer solution and in human whole saliva.

U.S. patent 4,564,519 describes a di-enzymatic chewable dentifrice which contains an oxidizable substrate and an oxidoreductase enzyme specific to such substrate for producing hydrogen peroxide upon chewing of the dentifrice and further contains a thiocyanate salt and lactoperoxidase for interacting with hydrogen peroxide to produce a hypothiocyanate bacterial inhibitor. The concentration of lactoperoxidase is at least about 2% of the concentration of the oxidoreductase enzyme, in International Units, to thereby limit the ratio of hydrogen peroxide to lactoperoxidase during oral chewing of the dentifrice. An illustrative enzymatic system for this purpose contains glucose, glucose oxidase, potassium thiocyanate and lactoperoxidase.

### Disclosure of the Invention

### Problems to be Solved by the Invention

As described above, keeping good oral hygiene is important to prevent periodontal disease and oral malodor. Particularly, oral care is very important for elderly persons and those who require nursing care to prevent aspiration pneumonia.

However, toothbrushing for oral care and spitting out toothpaste after toothbrushing by gargling are often very difficult and troublesome for elderly persons and those who require nursing care themselves and their caregivers. Particularly, failure in spitting out toothpaste may lead to aspiration accident and further cause aspiration pneumonia. For this reason, oral care for keeping good oral hygiene, such as toothbrushing, is highly risky for elderly persons and those who require nursing care depending on their conditions.

Therefore, there has been a demand for means for oral care for keeping good oral hygiene which do not require toothbrushing, gargling, and spitting out of toothpaste.

On the other hand, administering a strong synthetic disinfectant or the like approved only as a drug is effective for temporarily improving oral hygiene, but concern remains about its safety as a means for keeping good oral hygiene which is daily used for a long period of time such as several years or longer.

In view of these points, the present inventors have conceived an idea that all the above problems can be solved by using, as a means for oral care, a disinfectant which is safe, has few side-effects, can be consumed together with a food or drink by adding it to the food or drink, exhibits bactericidal effect in the oral cavity, and can be daily consumed for a long period of time without fear.

It is therefore an object of the present invention to provide a disinfectant which is safe, has few side-effects, can be consumed together with a food or drink by adding it to the food or drink, exhibits bactericidal effect in the oral cavity, and can be daily consumed for a long period of time without fear, and a food additive containing such a disinfectant.

In order to achieve the above object, the present inventors have extensively studied to find a disinfectant which can be added to foods and drinks, and as a result, have focused attention on a lactoperoxidase system containing lactoperoxidase, which is a milk protein. However, as described above, such a lactoperoxidase system requires, as an essential component, thiocyanate which is not approved as a food additive.

As a result of extensive study, the present inventors have found that a lactoperoxidase system containing components other than thiocyanate can exhibit strong bactericidal activity in human saliva by adding a pH adjusting component without thiocyanate or a thiocyanate substitute.

That is, the present inventors have found that the above object can be achieved by using, as an oral disinfectant, a system containing lactoperoxidase, which is a milk protein, glucose oxidase, glucose, and a pH adjusting component, and this finding has led to the completion of the present invention.

Further surprisingly, it has also been found that the oral disinfectant according to the present invention has antibacterial activity against Actinobacillus actinomycetemcomitans which is an oral bacterium conventionally believed to be less sensitive to a lactoperoxidase system.

The present invention includes the following (1) to (4).
(1) An oral disinfectant for killing Actinobacillus actinomycetemcomitans which comprises, as active ingredients, lactoperoxidase, glucose oxidase, glucose, and a pH adjusting component, wherein the pH of the disinfectant is adjusted to 4.4 to 5.2 by the pH adjusting component and wherein the disinfectant does not contain thiocyanate.
(2) The oral disinfectant according to the above (1), wherein the pH adjusting component is an organic acid and/or an organic acid salt.
(3) The oral disinfectant according to the above (2), wherein the organic acid is at least one acid selected from the group consisting of citric acid, lactic acid, malic acid, succinic acid, tartaric acid, and glutamic acid.
(4) A food additive or a pharmaceutical composition for prevention and/or treatment of periodontal disease, oral malodor, or aspiration pneumonia which contains the oral disinfectant according to any one of the above (1) to (3).

The disinfectant according to the present invention may be used to prepare:
(5) A food or drink containing the oral disinfectant according to any one of the above (1) to (3) or the food additive according to the above (4).
(6) A food or drink for prevention and/or treatment of periodontal disease, oral malodor, or aspiration pneumonia which contains the oral disinfectant according to any one of the above (1) to (3) or the food additive according to the above (4).

### Effect of the Invention

The oral disinfectant or food additive according to the present invention is effective as a means for keeping good oral hygiene, and is further safe, has few side-effects, can be consumed together with a food or drink by adding it to the food or drink, exhibits bactericidal effect in the oral cavity, and can be daily consumed for a long period of time without fear.

Further, oral care using the oral disinfectant or food additive according to the present invention does not require elderly persons and those who require nursing care to perform activities, such as toothbrushing and spitting out toothpaste after toothbrushing by gargling, which are not only very difficult and troublesome for these persons themselves and their caregivers but also highly risky depending on their conditions.

Further, the oral disinfectant and food additive according to the present invention can provide the following effects.
(1) Oral bacteria can be effectively killed.
(2) Diseases caused by oral bacteria can be effectively prevented and/or treated.
(3) Daily consumption is possible due to their safety for humans.
(4) Foods and drinks having the effect of preventing periodontal disease etc. can be provided by adding them to foods and drinks.

### Brief Description of the Drawings

Fig. 1 is a graph showing a change in hydrogen sulfide content in human exhaled breath caused by administration of the oral disinfectant according to the present invention; and
Fig. 2 is a graph showing a change in volatile sulfur compound content in human exhaled breath caused by administration of the oral disinfectant according to the present invention.

### Best Mode for Carrying out the Invention

Hereinbelow, a preferred embodiment of the present invention will be described in detail. However, the present invention is not limited to the following preferred embodiment, and can be arbitrarily changed within the scope thereof. It is to be noted that in this specification, "%" represents "% by mass" unless otherwise specified.

Lactoperoxidase to be used in the present invention can be obtained from the milk or the like of mammals such as humans, cows, horses, sheep, and goats. It is preferred that the lactoperoxidase is industrially produced from unheated whey from milk or the like or skimmed milk by a conventional method (e.g., ion-exchange chromatography) such as a method disclosed in, for example, Japanese Patent Application Laid-Open No. 05-41981 (title of the invention: Viable Cell-Containing Liquid Composition). Alternatively, it is also possible to use commercially-available natural product-derived lactoperoxidase (e.g., produced by Biopole) or recombinant lactoperoxidase (e.g., recombinant lactoperoxidase expressed and purified by a method described by Shin et al. ("Biochemical and Biophysical Research Communications", vol. 271, 2000, pp. 831-836) or commercially-available recombinant lactoperoxidase).

In the present invention, lactoperoxidase derived from the milk of mammals is preferably used. As lactoperoxidase to be used for oral disinfectants, food additives, and pharmaceutical compositions, one derived from the milk of cows, sheep, or goats traditionally used for foods and drinks is preferably used, and one derived from cow's milk is particularly preferably used. This is because such milk have been historically used for foods and drinks for humans for a long period of time, and therefore their extremely high level of safety for humans is ensured.

Further, unheated whey derived from cow's milk can be stably obtained in a large amount as a by-product of the manufacture of milk products, and is therefore particularly preferably used as a raw material of lactoperoxidase to be used in the present invention.

As glucose oxidase to be used in the present invention, for example, commercially-available glucose oxidase (e.g., produced by SHINNIHON-KAGAKU-KOGYO) which is an enzyme produced by a microorganism such as Aspergillus niger or Penicillium chrysogenum can be used.

As glucose to be used in the present invention, for example, commercially-available glucose for food additives (e.g., produced by NIHON SHOKUHIN KAKO Co., Ltd.) can be used.

A pH adjusting component to be used in the present invention is not particularly limited as long as it has a pH buffering ability to keep the oral disinfectant weak acidic (i.e., pH 4.4 to 5.2) when the oral disinfectant is suspended in a solvent such as saliva. For example, an organic acid and/or an organic acid salt can be used. For example, it is possible to use a combination of at least one acid selected from the group consisting of citric acid, lactic acid, malic acid, succinic acid, tartaric acid, and glutamic acid which are commercially-available food additives and at least one salt selected from the group consisting of citric acid salts (e.g., trisodium citrate, tripotassium citrate), lactic acid salts (e.g., sodium lactate), malic acid salts (e.g., sodium malate), succinic acid salts (e.g., monosodium succinate, disodium succinate), tartaric acid salts (e.g., sodium tartrate, potassium hydrogen tartrate), and glutamic acid salts (e.g., sodium glutamate, potassium glutamate).

All these glucose oxidase, glucose, organic acids, and salts are widely used as food additives and generally sold, and are therefore easily available.

When lactoperoxidase, glucose oxidase, glucose, an organic acid, and a salt are mixed with saliva, the mixture has the effect of killing oral bacteria, and therefore they can be used as active ingredients of an oral disinfectant (hereinafter, also referred to as "disinfectant according to the present invention", "disinfectant composition according to the present invention", or "composition according to the present invention"). The term "active ingredient" used herein means an ingredient contributing to the effect of killing oral bacteria. The disinfectant according to the present invention may further contain an ingredient having the effect of killing oral bacteria other than ingredients involved in a lactoperoxidase system. For example, the disinfectant according to the present invention may contain a useful milk protein other than lactoperoxidase, such as lactoferrin, lysozyme, immunoglobulin, casein, α-lactalbumin, or β-lactoglobulin, and/or a lactic acid bacterium that acts as probiotics.

The oral disinfectant according to the present invention has the effect of killing oral bacteria. Therefore, the disinfectant according to the present invention can be used as, for example, a disinfectant having the effect of preventing and treating various diseases caused by oral bacteria, such as periodontal disease, oral malodor, and aspiration pneumonia. However, the use of the oral disinfectant according to the present invention is not limited to prevention and/or treatment of diseases caused by oral bacteria.

The oral disinfectant according to the present invention contains, as its active ingredients, food materials and food additives such as milk proteins, enzymes, saccharides, acids, and salts, and is therefore very safe for humans. Further, the oral disinfectant according to the present invention exhibits the effect of preventing and/or treating diseases caused by oral bacteria when it is orally consumed on a daily basis. Examples of the diseases caused by oral bacteria include periodontal disease, oral malodor, and aspiration pneumonia.

The oral disinfectant according to the present invention may be constituted from only a mixture of lactoperoxidase, glucose oxidase, glucose, an acid, and a salt, or may further contain an ingredient other than that. The ingredient other than lactoperoxidase, glucose oxidase, glucose, an acid, and a salt, can be appropriately selected according to the form of consumption of the oral disinfectant. For example, the ingredient other than lactoperoxidase, glucose oxidase, glucose, an acid, and a salt can be appropriately used according to administration route such as oral administration. The oral disinfectant according to the present invention can also be processed into tablets, capsules, troches, syrups, granules, and powders by well known methods.

The oral disinfectant according to the present invention can be produced as, for example, a formulation containing, as active ingredients, lactoperoxidase, glucose oxidase, glucose, and a pH adjusting component by using any pharmaceutically-acceptable additives such as excipients. A food additive according to the present invention may also be produced as a formulation. In the case of producing such a formulation, the total amount of active ingredients contained in the formulation is usually in the range of 0.005 to 20% by mass, preferably in the range of 0.05 to 12.5% by mass. For production of the formulation, additives such as excipients, binders, disintegrants, lubricants, stabilizers, flavoring agents, diluents, and injection solvents can be used.

Examples of the excipients include: saccharide derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethylcellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate. Examples of the binders include, in addition to the above excipients, gelatin; polyvinylpyrrolidone; and macrogol. Examples of the disintegrants include, in addition to the above excipients, chemically-modified starch or cellulose derivatives such as croscarmellose sodium, sodium carboxymethylstarch, and cross-linked polyvinylpyrrolidone. Examples of the lubricants include: talc; stearic acid; metal stearates such as calcium stearate and magnesium stearate; colloidal silica; waxes such as bee gum and spermaceti wax; boric acid; glycol; carboxylic acids such as fumaric acid and adipic acid; sodium carboxylates such as sodium benzoate; sulfate salts such as sodium sulfate; leucine; lauryl sulfates such as sodium lauryl sulfate and magnesium lauryl sulfate; silicic acids such as anhydrous silicic acid and silicic acid hydrate; and starch derivatives. Examples of the stabilizers include: p-hydroxybenzoate esters such as methylparaben and propylparaben; alcohols such as chlorobutanol, benzyl alcohol, and phenylethyl alcohol; benzalkonium chloride; anhydrous acetic acid; and sorbic acid. Examples of the flavoring agents include sweeteners, acidulants, and fragrant materials. Examples of the injection solvents include water, ethanol, and glycerin.

The oral disinfectant or food additive according to the present invention can also be administered by adding it to a food or drink. The dosage and the number of doses vary depending on intended effect, administration route, duration of therapy, age, and body weight, etc. However, in general, the dosage can be appropriately selected from a range of 10 mg to 10 g per day per adult, and the number of doses can be appropriately selected from a range of once to several times per day. The length of administration is preferably 7 days or longer.

Examples of foods and drinks containing the oral disinfectant or food additive according to the present invention containing lactoperoxidase, glucose oxidase, glucose, an acid, and a salt include: soft drinks, milk drinks, and concentrated stock solutions and conditioning powders of these drinks; milk products such as processed milk and fermented milk; enteral foods and conditioning powders thereof and thickening foods; and functional foods. Other examples of such foods and drinks include: drinks such as carbonated drinks, energy drinks, and fruit drinks (including stock solutions and conditioning powders of these drinks); frozen desserts such as ice creams, sorbets, and ice shavings topped with syrups; noodles such as Japanese noodles of buckwheat, Japanese noodles of wheat, bean-starch noodles, pasta-wrappings for Chinese stuffed dumplings, pasta-wrappings for Chinese steamed dumplings, Chinese noodles, and instant noodles; confectionery such as drops, chewing gums, candies, gums, chocolates, tablets, snack foods, biscuits, jellies, jams, creams, and baked goods; processed fish or meat products such as Japanese steamed fish pastes, hams, and sausages; fats and oils and oil processed foods such as salad oils, frying oils, margarine, mayonnaise, shortening, whipped cream, and dressing; seasonings such as sauces; and soups, stews, salads, prepared foods, pickles, and breads. These foods and drinks can be produced by, for example, blending the oral disinfectant or food additive according to the present invention (including its powder or an aqueous solution of the powder (e.g., syrup)) with saccharides such as dextrin and starch; proteins such as gelatin, soybean protein, and corn protein; amino acids such as alanine, glutamine, and isoleucine; polysaccharides such as cellulose and gum arabic; and/or oils and fats such as soybean oil and medium-chain triglyceride.

As a preferred form of such foods and drinks, a supplement in tablet form can be mentioned by way of example. In this case, it is possible to accurately know the amount of active ingredients consumed and the calories consumed by taking active ingredients.

As another preferred form of such foods and drinks, a liquid, especially a thickened liquid can be mentioned. This is because a liquid spreads throughout the oral cavity, and particularly, can easily enter spaces between dental hard tissue and gingival tissue where periodontal disease occurs, and a thickened liquid can retain in the oral cavity for a longer time and therefore bactericidal effect can be more effectively exhibited. For this reason, according to the present invention, a preferred form of the formulation, food, and drink described above is a liquid, especially a thickened liquid.

It is to be noted that the oral disinfectant or food additive according to the present invention can be blended with foods and drinks having an indication of their efficacy in prevention or treatment of the diseases described below. That is, it is possible to indicate that such foods and drinks and the foods and drinks according to the present invention are intended for prevention or treatment of diseases caused by oral bacteria, such as periodontal disease, oral malodor, and aspiration pneumonia.

The term "indication" used herein means an action to inform consumers of the efficacy described above. Examples of such an action include an action to indicate the efficacy on the products of foods and drinks containing the oral disinfectant or food additive according to the present invention or the foods and drinks according to the present invention or on the packages, advertisements, and the like of the products and an action to transfer, deliver, and display the products having an indication of the efficacy. Particularly, a preferred embodiment thereof is an indication as food for specified health use (see Enforcement Regulations of Health Promotion Law (Japan Ministry of Health, Labour, and Welfare, Ministerial ordinance No. 86, April 30, 2003) Article 12, Section 1, Item 5).

### EXAMPLES

The present invention will be described in detail with reference to the following examples. The present invention is not limited to these examples.

As described above, the present invention has been completed as a result of an extensive study and based on the findings that a lactoperoxidase system containing only components other than thiocyanate exhibits strong bactericidal activity in human saliva by adding a pH adjusting component without thiocyanate or a thiocyanate substitute.

The present inventors have studied a mechanism by which the oral disinfectant according to the present invention exhibits its bactericidal activity, and have reached the idea that the existence of a trace amount of thiocyanate in saliva may contribute to the exhibition of antibacterial activity. Based on the idea, the following experiments were carried out, and as a result it has been confirmed that the effect of the oral disinfectant according to the present invention is reliably reproduced.

In order to evaluate the bactericidal activity of the oral disinfectant according to the present invention against oral bacteria, the bactericidal activity of the oral disinfectant against Actinobacillus actinomycetemcomitans, which is a human oral bacterium reported as being less sensitive to a lactoperoxidase system activated by a combination use of lactoperoxidase, hydrogen peroxide, and thiocyanate (International Journal of Antimicrobial Agents, Netherlands, vol. 21, 2003, pp. 434-440) was examined. It is to be noted that, as a solvent, a human saliva sample was used or a phosphate buffered saline solution containing 0.5 mM thiocyanate to achieve the same thiocyanate level as saliva was used as a substitute for saliva. The details will be described with reference to the following test examples.
Hereinbelow, the effect of the oral disinfectant according to the present invention will be described in detail with reference to the following test examples.

### <Test Example 1 >

This test was carried out to examine the effect of the oral disinfectant on oral bacteria.

### (1) Preparation of Samples

15 g of erythritol powder (produced by Nikken Chemicals Co., Ltd.), 52.5 g of reduced maltose syrup powder (produced by Towa Chemical Industry Co., Ltd.), 30 g of sorbitol powder (produced by Towa Chemical Industry Co., Ltd.), 15 g of corn starch powder (produced by Oji Cornstarch Co., Ltd.), 0.75 g of acerola flavor powder (produced by Takasago International Corporation), 6 g of sucrose fatty acid ester powder (produced by Mitsubishi-Kagaku Foods Corporation), 2.58 g of citric acid monohydrate powder (produced by Kokusan Chemical Co., Ltd.), 5.21 g of trisodium citrate dehydrate powder (produced by Kokusan Chemical Co., Ltd.), 18 g of xylitol powder (produced by Wako Pure Chemical Industries, Ltd.), 4.5 g of glucose powder (produced by Wako Pure Chemical Industries, Ltd.), 0.05 g of lactoperoxidase powder (produced by Biopole), and 0.4 g of glucose oxidase powder (produced by Shinnihon-Kagaku-Kogyo) were uniformly mixed in a mortar to prepare a powder mixture as a test sample. As a control sample, a powder mixture was prepared in the same manner as the test sample except that lactoperoxidase was not added.

### (2) Preparation of Cell Culture Solution

Actinobacillus actinomycetemcomitans JCM 8578 (obtained from Riken) was cultured overnight in a brain heart infusion liquid medium (produced by Becton, Dickinson and Company) to prepare a cell culture solution.

### (3) Test Method

0.5 g of the test sample or control sample was added to a 50 mL sterile disposable tube. Then, 3.3 mL of a phosphate buffered saline solution containing 0.5 mM sodium thiocyanate to achieve the same thiocyanate level as human saliva was added to the tube to suspend the test sample or control sample in the phosphate buffered saline solution. Then, 33.3 µL of the cell culture solution prepared in the above (2) was added to the suspension, and the thus obtained mixture was incubated in an incubator filled with 10% carbon dioxide gas at 37°C for 15 minutes under stirring. The mixture was serially diluted 10-fold with a phosphate buffered saline solution and then inoculated on a trypticase soy agar medium allowing Actinobacillus actinomycetemcomitans to selectively grow by addition of 10% horse serum, 0.1 % yeast extract, 75 µg/mL bacitracin, and 5 µg/mL vancomycin (Journal of Clinical Microbiology, America, vol. 15, 1982, pp. 606-609), and incubated in an incubator filled with 10% carbon dioxide gas at 37°C for 3 days. After the incubation, the number of colonies formed on the agar medium was counted. From the number of colonies of Actinobacillus actinomycetemcomitans, the logarithm of the number of viable cells per milliliter of the suspension (log₁₀ cfu/mL) was determined.

### (4) Test result

The result of the test is shown in Table 1.

**[Table 1]**

| Sample | Number of cells of *A. actinomycetemcomitans* (log₁₀ cfu/ml) |
|---|---|
| Test sample | N.D. |
| Control sample | 6.82 |

| | |
|---|---|
| N.D. : below detection limit (<1.70) Initial number of cells : 7.0 log₁₀ cfu/ml | |

As can be seen from the result, the test sample containing lactoperoxidase, glucose oxidase, glucose, an acid, and a salt reduced the number of viable cells of Actinobacillus actinomycetemcomitans to below the detection limit, that is, showed a strong bactericidal effect. On the other hand, in the case of the control sample not containing lactoperoxdidase, the number of viable cells was hardly changed. It is to be noted that another test was carried out in the same manner as in Example 1 except that the phosphate buffered saline solution containing sodium thiocyanate was changed to filter-sterilized saliva sampled from a healthy adult. As a result, the test sample showed strong bactericidal activity.

### <Test Example 2>

This test was carried out to examine the influence of a pH adjusting agent and glucose on the effect of the oral disinfectant on oral bacteria.

### (1) Preparation of Sample

15 g of erythritol powder (produced by Nikken Chemicals Co., Ltd.), 52.5 g of reduced maltose syrup powder (produced by Towa Chemical Industry Co., Ltd.), 30 g of sorbitol powder (produced by Towa Chemical Industry Co., Ltd.), 15 g of corn starch powder (produced by Oji Cornstarch Co., Ltd.), 0.75 g of acerola flavor powder (produced by Takasago International Corporation), 6 g of sucrose fatty acid ester powder (produced by Mitsubishi-Kagaku Foods Corporation), 18 g of xylitol powder (produced by Wako Pure Chemical Industries, Ltd.), 0.05 g of lactoperoxidase powder (produced by Biopole), and 0.4 g of glucose oxidase powder (produced by Shinnihon-Kagaku-Kogyo) were uniformly mixed in a mortar to prepare a powder mixture not containing glucose, an acid, and a salt.

### (2) Preparation of Cell Culture Solution

A cell culture solution of Actinobacillus actinomycetemcomitans was prepared in the same manner as in Test Example 1.

### (3) Test method

0.459 g of the powder mixture prepared in the above (1) not containing a pH adjusting agent and glucose was added to a 50 mL sterile disposable tube. Then, 1.11 mL of a 3-fold concentration phosphate buffer solution, 1.0 mL of a 100 mM citrate buffer solution (pH 4.6, 5.0, 5.4, 5.8, or 6.2) or 100 mM phosphate buffer solution (pH 6.6, 7.0, or 7.4), and 33.3 µL of 50 mM sodium thiocyanate were added to the tube, and a 15 mg/mL aqueous glucose solution and purified water were further added so that the final glucose concentration was 0, 0.045, 0.15, 0.45, 1.5, or 4.5 mg/mL and the total amount of the solutions added was 3.3 mL to obtain a suspension. Then, 33.3 µL of the cell culture solution prepared in the above (2) was added to the suspension, and the thus obtained mixture was incubated in an incubator filled with 10% carbon dioxide gas at 37°C for 15 minutes under stirring. Then, the number of viable cells of Actinobacillus actinomycetemcomitans contained in the mixture was measured in the same manner as in Test Example 1. In this test, when the number of viable cells was reduced to one-tenth or less of the initial number of cells, bactericidal activity was evaluated as effective.

### (4) Test result

The result of the test is shown in Table 2.

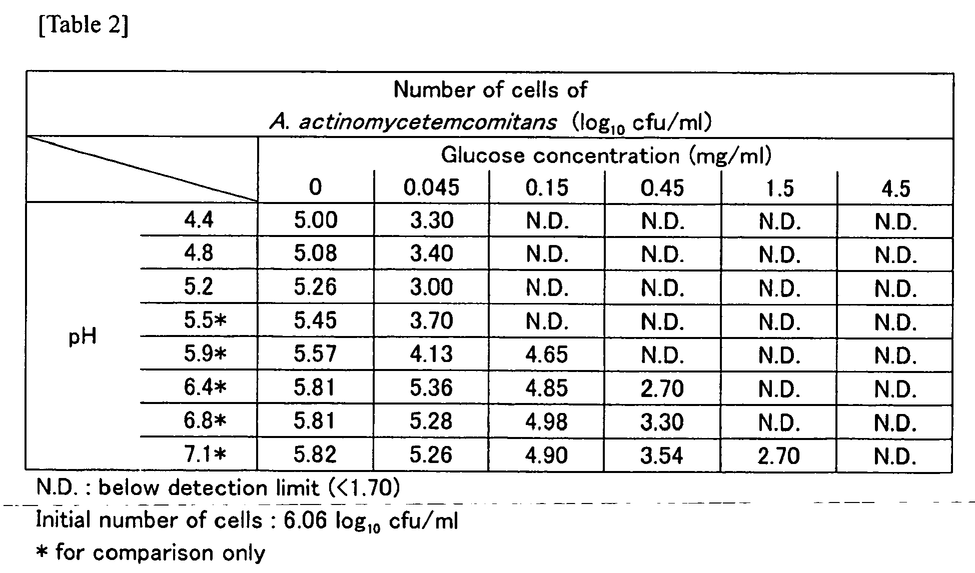

The pH values shown in Table 2 are the measured pH values of the suspensions each containing the buffer solution having a certain pH. In Table 2, the rows represent the results obtained at different pH values and the columns represent the results obtained at different glucose concentrations. As can be seen from the result, when the measured pH value was 5.9 or less (i.e., under weak acidic conditions) and glucose was added, the test sample had effective bactericidal activity irrespective of the concentration of glucose. From the result, it has become clear that the oral disinfectant exhibits strong bactericidal activity when it contains an acid and a salt which have a buffering ability to keep the pH of the suspension of the oral disinfectant in a solvent at 5.9 or less. Such bactericidal activity was effectively exhibited at a pH of preferably 5.9 or less. More specifically, in a case where the concentration of glucose was 0.15 mg/mL or more, by keeping pH at 5.5 or less, bactericidal activity was so effectively exhibited that the number of viable cells was below the detection limit. In a case where the concentration of glucose was 0.45 mg/mL or more, by keeping pH at 5.9 or less, bactericidal activity was so effectively exhibited that the number of viable cells was below the detection limit. In a case where the concentration of glucose was 1.5 mg/mL or more, by keeping pH at 6.8 or less, bactericidal activity was so effectively exhibited that the number of viable cells was below the detection limit. In a case where the concentration of glucose was 4.5 mg/mL or more, by keeping pH at 7.1 or less, bactericidal activity was so effectively exhibited that the number of viable cells was below the detection limit. Further, in a case where the concentration of glucose was 0.15 mg/mL or more, bactericidal activity was effectively exhibited at any pH within the range of 4.4 to 7.1. From the result, the amount of glucose that should be contained in the oral disinfectant was determined by calculation. As a result, it has become clear that whichever of the buffer solutions described above is used as an acid and a salt, a strong bactericidal effect can be obtained as long as a glucose content in the composition (i.e., a percentage of the weight of glucose with respect to the total weight of the mixture, glucose, an acid, and a salt) is 0.1% or more.

From the above result, it has become clear that the oral disinfectant according to the present invention exhibits its bactericidal activity by utilizing a trace amount of thiocyanate contained in saliva. Further, it has also been found that the oral disinfectant according to the present invention has bactericidal activity also against Actinobacillus actinomycetemcomitans previously reported as being less sensitive to the bactericidal activity of a lactoperoxidase system.

As described above, the oral disinfectant according to the present invention does not contain a thiocyanate component therein and does not require external addition of thiocyanate, but exhibits sufficient bactericidal activity by utilizing a trace amount of thiocyanate contained in saliva. Therefore, the oral disinfectant according to the present invention is a disinfectant which can be used without any fear also by elderly persons who are more likely to take the wrong amount of a drug and those who require nursing care. Such an oral disinfectant is very safe also as a food additive.

Further, as described above, the oral disinfectant according to the present invention exhibits bactericidal activity against Actinobacillus actinomycetemcomitans, and therefore the present invention provides a novel oral disinfectant against Actinobacillus actinomycetemcomitans.

Hereinbelow, the present invention will be described in more detail with reference to the following production example (Example 1), but is not limited to this production example.

### Example 1

150 g of erythritol powder (produced by Nikken Chemicals Co., Ltd.), 525 g of reduced maltose syrup powder (produced by Towa Chemical Industry Co., Ltd.), 300 g of sorbitol powder (produced by Towa Chemical Industry Co., Ltd.), 150 g of corn starch powder (produced by Oji Cornstarch Co., Ltd.), 7.5 g of acerola flavor powder (produced by Takasago International Corporation), 60 g of sucrose fatty acid ester powder (produced by Mitsubishi-Kagaku Foods Corporation), 25.8 g of citric acid powder (produced by San-Ei Gen F.F.I., Inc.), 52.1 g of trisodium citrate powder (produced by San-Ei Gen F.F.I., Inc.), 180.1 g of xylitol powder (produced by Towa Chemical Industry Co., Ltd.), 45 g of glucose powder (produced by Nihon Shokuhinkako Co., Ltd.), 0.5 g of lactoperoxidase powder (produced by Biopole), and 4 g of glucose oxidase powder (produced by Shinnihon-Kagaku-Kogyo) were uniformly mixed to obtain a powder mixture, and the powder mixture was continuously tableted using a tablet machine (manufactured by Hata Iron Works Co., Ltd) at a tableting speed of 12 tablets/min at a pressure of 9.8 KPa to obtain 1800 oral disinfectant tablets (about 900 g) each having a mass of 0.5 g.

### <Test Example 3>

This test was carried out to examine the effect of the oral disinfectant according to the present invention on human breath odor.

### (1) Preparation of Sample

### (Test Sample)

Tablets (i.e., the oral disinfectant according to the present invention) produced in the same manner as in Example 1 except that the mass of each tablet was changed to 0.6 g were used as a test sample.

### (Control Sample)

Tablets produced in the same manner as in Example 1 except that lactoperoxidase and glucose oxidase were replaced with reduced maltose syrup and that the mass of each tablet was changed to 0.6 g were used as a control sample. It is to be noted that the composition of the components other than lactoperoxidase and glucose oxidase of the control sample was the same as that of the test sample.

### (2) Test Method

### (Study Group)

Three healthy subjects prohibited from tooth cleaning such as toothbrushing and gargling, drinking, and eating after wake-up on a test day continuously took 3 test sample tablets by dissolving them one by one in the oral cavity in the morning on the test day (study group). Their exhaled breath was sampled from the oral cavity before taking tablets and after a lapse of 1 hour and a lapse of 2 hours from taking tablets to measure the amount of change in hydrogen sulfide (known as a substance responsible for breath odor) content and the amount of change in volatile sulfur compound content (i.e., the total amount of change in hydrogen sulfide, methyl mercaptan, and dimethyl sulfide contents) by a oral malodor analyzer ("Oral Chroma" produced by Abilit Corporation). The results of the three subjects were averaged and expressed in nanograms (ng) per 10 mL of exhaled breath.

### (Control Group)

A control test was carried out in the following manner on a different day from the test day of the study group. Three healthy subjects prohibited from tooth cleaning such as toothbrushing and gargling, drinking, and eating after wake-up on a test day took 3 control sample tablets (not containing lactoperoxidase and glucose oxidase) in the same manner as in the study group, and then their exhaled breath was sampled from the oral cavity and measured by a oral malodor analyzer (control group).

### (No-tablet group)

Further, on a different day from the test day of the study group and the test day of the control group, three healthy subjects prohibited from tooth cleaning such as toothbrushing and gargling, drinking, and eating after wake-up on a test day took no tablets, and their exhaled breath was sampled from the oral cavity to measure the amount of change in hydrogen sulfide content and the amount of change in volatile sulfur compound content by a oral malodor analyzer (no-tablet group).

### (3) Test Result

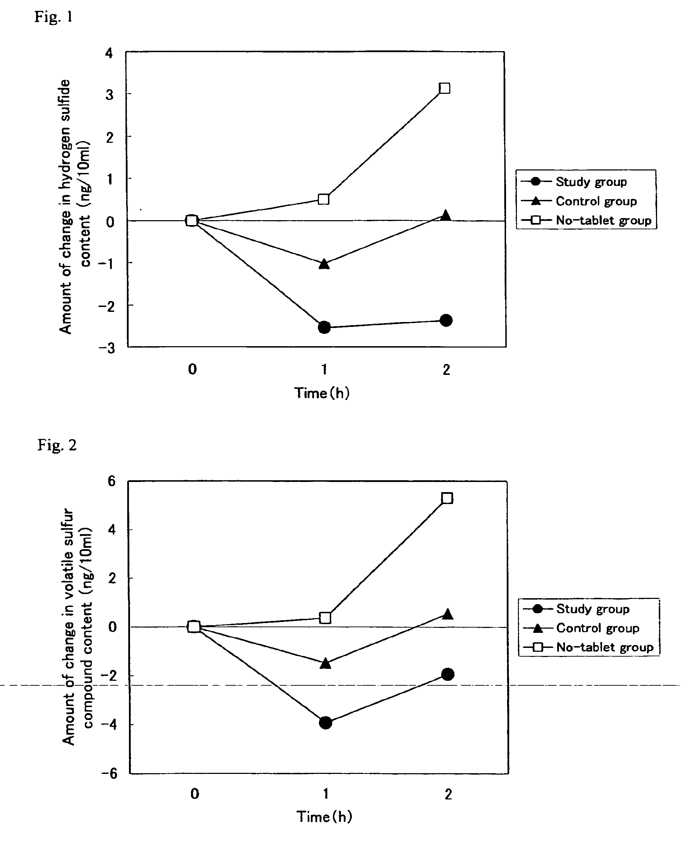

The result of Test Example 3 is shown in Tables 3 and 4. Table 3 shows the amount of change in hydrogen sulfide content in exhaled breath of each group, and Table 4 shows the amount of change in volatile sulfur compound content in exhaled breath of each group. Fig. 1 shows a graph of Table 3, and Fig. 2 shows a graph of Table 4.

**[Table 3]**

| Time (h) | Amount of change in hydrogen sulfide content (ng/10ml) | | |
|---|---|---|---|
| | Study group | Control group | No-tablet group |
| 0 | 0.00 | 0.00 | 0.00 |
| 1 | -2.54 | -1.02 | 0.50 |
| 2 | -2.37 | 0.13 | 3.14 |
| | | | |

**[Table 4]**

| Time(h) | Amount of change in volatile sulfur compound content (ng/10ml) | | |
|---|---|---|---|
| | Study group | Control group | No-tablet group |
| 0 | 0.00 | 0.00 | 0.00 |
| 1 | -3.93 | -1.48 | 0.37 |
| 2 | -1.94 | 0.55 | 5.30 |

### a) Amount of change in hydrogen sulfide content

As can be seen from Table 3 and Fig. 1, in a case where the subjects took no tablets (i.e., No-tablet group indicated by the symbol "□" in Fig. 1), the hydrogen sulfide content of exhaled breath measured after 1 hour was slightly increased, and at this time, the amount of change in hydrogen sulfide content was 0.50 ng/10 mL, and the amount of change in hydrogen sulfide content in exhaled breath measured after 2 hours reached 3.14 ng/10 mL. On the other hand, in a case where the subjects took the tablets according to the present invention (i.e., Study group indicated by the symbol "●" in Fig. 1), the hydrogen sulfide content of exhaled breath measured after 1 hour was lower than that measured before taking tablets by 2.54 ng/10 mL, and the hydrogen sulfide content of exhaled breath measured after 2 hours was higher than that measured after 1 hour by 0.17 ng/10 mL but was kept lower than that measured before taking tablets by 2.37 ng/10 mL. In the case of the group taking tablets not containing lactoperoxidase and glucose oxidase (i.e., Control group indicated by the symbol "▲" in Fig. 1), the hydrogen sulfide content of exhaled breath measured after 1 hour was once reduced by 1.02 ng/10 mL from that measured before taking tablets, but after 2 hours, the hydrogen sulfide content of exhaled breath was returned to a level similar to that before taking tablets. From the result, it has been confirmed that the tablets not containing lactoperoxidase and glucose oxidase do not have the effect of keeping the hydrogen sulfide content of exhaled breath at a low level. More specifically, after 1 hour from taking tablets, the study group showed an about 2.5-fold decrease in the hydrogen sulfide content of exhaled breath as compared to the control group. Further, even after 2 hours from taking tablets, in the study group, the hydrogen sulfide content of exhaled breath was kept at a low level similar to that measured after 1 hour from taking tablets, whereas in the control group, a change in the hydrogen sulfide content of exhaled breath shifted from a decrease to an increase.

### b) Amount of change in volatile sulfur compound content (Total amount of change in hydrogen sulfide, methyl mercaptan, and dimethyl sulfide contents)

As can be seen from Table 4 and Fig. 2, in a case where the subjects took no tablets (i.e., No-tablet group indicated by the symbol "□" in Fig. 2), the volatile sulfur compound content of exhaled breath measured after 1 hour was slightly increased, and at this time, the amount of change in volatile sulfur compound content was 0.37 ng/10 mL, and the amount of change in volatile sulfur compound content in exhaled breath measured after 2 hours reached 5.30 ng/10 mL. On the other hand, in a case where the subjects took the tablets according to the present invention (i.e., Study group indicated by the symbol "●" in Fig. 2), the volatile sulfur compound content of exhaled breath measured after 1 hour was lower than that measured before taking tablets by 3.93 ng/10 mL, and the volatile sulfur compound content of exhaled breath measured after 2 hours was higher than that measured after 1 hour by 1.99 ng/10 mL but was kept lower than that measured before taking tablets by 1.94 ng/10 mL. In the case of the group taking tablets not containing lactoperoxidase and glucose oxidase (i.e., Control group indicated by the symbol "▲" in Fig. 2), the volatile sulfur compound content of exhaled breath measured after 1 hour was once reduced by 1.48 ng/10 mL from that measured before taking tablets, but after 2 hours, the volatile sulfur compound content of exhaled breath was returned to a level similar to that before taking tablets. From the result, it has been confirmed that the tablets not containing lactoperoxidase and glucose oxidase do not have the effect of keeping the volatile sulfur compound content of exhaled breath at a low level. More specifically, after I hour from taking tablets, the study group showed an about 2.6-fold decrease in the volatile sulfur compound content of exhaled breath as compared to the control group. Further, even after 2 hours from taking tablets, in the study group, the volatile sulfur compound content of exhaled breath was kept at a low level about half of that measured after 1 hour from taking tablets, whereas in the control group, a change in the volatile sulfur compound content of exhaled breath shifted from a decrease to an increase.

As described above, it has been found that the hydrogen sulfide content and total volatile sulfur compound content of exhaled breath sampled from the oral cavity is decreased by taking the oral disinfectant according to the present invention containing lactoperoxidase and glucose oxidase (i.e., Study group) and it has been also found that the oral disinfectant according to the present invention has the effect of keeping the hydrogen sulfide content and total volatile sulfur compound content of exhaled breath at a low level for a certain period of time. That is, it has been found that the oral disinfectant according to the present invention exhibits its bactericidal activity not only in an in-vitro experimental system but also in an actual human oral cavity so that components causing breath odor contained in human exhaled breath are reduced (i.e., the oral disinfectant according to the present invention actually has the effect of reducing human breath odor) and such an effect can be obtained without any side-effects by simply administering the oral disinfectant according to the present invention in a human oral cavity.

### Industrial Applicability

The oral disinfectant or food additive according to the present invention is an effective means for keeping good oral hygiene, and is further safe, has few side-effects, can be consumed together with a food or drink by adding it to the food or drink, exhibits bactericidal activity in the oral cavity, and can be daily consumed for a long period of time without fear.

## Claims

1. An oral disinfectant for killing Actinobacillus actinomycetemcomitans which comprises, as active ingredients, lactoperoxidase, glucose oxidase, glucose, and a pH adjusting component, wherein the pH of the disinfectant is adjusted to 4.4 to 5.2 by the pH adjusting component and wherein the disinfectant does not contain thiocyanate.

2. The oral disinfectant according to claim 1, wherein the pH adjusting component is an organic acid and/or an organic acid salt.

3. The oral disinfectant according to claim 2, wherein the organic acid is at least one acid selected from the group consisting of citric acid, lactic acid, malic acid, succinic acid, tartaric acid, and glutamic acid.

4. A food additive or a pharmaceutical composition for use in prevention and/or treatment of periodontal disease, oral malodor, or aspiration pneumonia, comprising the oral disinfectant according to any one of claims 1 to 3.

## Patentansprüche

1. Orales Desinfektionsmittel zum Abtöten von Actinobacillus actinomycetemcomitans, das als aktive Ingredienzien Laktoperoxidase, Glucoseoxidase, Glucose und eine den pH einstellende Komponente umfasst, wobei der pH des Desinfektionsmittels durch die den pH einstellende Komponente auf 4,4 bis 5,2 eingestellt ist und wobei das Desinfektionsmittel kein Thiocyanat enthält.

2. Orales Desinfektionsmittel gemäß Anspruch 1, wobei die den pH einstellende Komponente eine organische Säure und/oder ein Salz einer organischen Säure ist.

3. Orales Desinfektionsmittel gemäß Anspruch 2, wobei die organische Säure wenigstens eine Säure ist, die ausgewählt ist aus der Gruppe, bestehend aus Citronensäure, Milchsäure, Äpfelsäure, Bersteinsäure, Weinsäure und Glutaminsäure.

4. Nahrungsmitteladditiv oder pharmazeutische Zusammensetzung zur Verwendung bei der Prävention und/oder Behandlung einer Zahnfleischerkrankung, von Mundgeruch oder Aspirationspneumonie, das/die das orale Desinfektionsmittel gemäß einem der Ansprüche 1 bis 3 umfasst.

## Revendications

1. Désinfectant oral pour tuer Actinobacillus actinomycetemcomitans qui comprend, comme ingrédients actifs, de la lacto-peroxydase, de la glucose oxydase, du glucose et un composant ajustant le pH, dans lequel le pH du désinfectant est ajusté à 4,4 à 7,2 par le composant ajustant le pH et dans lequel le désinfectant ne contient pas du thiocyanate.

2. Désinfectant oral selon la revendication 1, dans lequel le composant ajustant le pH est un acide organique et/ou un sel d'un acide organique.

3. Désinfectant oral selon la revendication 2, dans lequel ledit acide organique est un acide choisi parmi les groupes consistant en acide citrique, acide lactique, acide malique, acide succinique, acide tartrique et acide glutamique.

4. Additif alimentaire ou composition pharmaceutique destiné(e) à l'utilisation pour la prévention et/ou le traitement de la maladie périodontique, de la mauvaise haleine ou de la pneumonie de déglutition, refermant le désinfectant oral selon l'une quelconque des revendications 1 à 3.
